# EUROPÄISCHE PATENTSCHRIFT

(11) **EP 0 701 995 B1**
(45) Veröffentlichungstag und Bekanntmachung des Hinweises auf die Patenterteilung: **23.12.1998**
(21) Anmeldenummer: 95112478.3
(22) Anmeldetag: 09.08.1995
(51) Int. Cl.: C07C 209/68, C07C 209/72

(54) **Verfahren zur Herstellung von aromatischen Aminen aus aliphatischen Aminen sowie deren Herstellung durch aminierende Hydrierung**
Process for the preparation of aromatic amines from aliphatic amines and the preparation of the latter by amino-hydrogenation
Procédé de préparation d'amines aromatiques à partir d'amines aliphatiques et la préparation de celles-ci par amino-hydrogénation

(30) Priorität: 16.08.1994 DE 4429014
(43) Veröffentlichungstag der Anmeldung: 20.03.1996
(73) Patentinhaber: BASF AKTIENGESELLSCHAFT, 67056 Ludwigshafen (DE)
(72) Erfinder: Köhler, Ulrich, Dr., D-68259 Mannheim (DE); Pape, Frank-Friedrich, Dr., D-67259 Kleinniedesheim (DE); Irgang, Matthias, Dr., D-69121 Heidelberg (DE); Wulff-Döring, Joachim, Dr., D-67227 Frankenthal (DE); Hesse, Michael, Dr., D-67105 Schifferstadt (DE); Polanek, Peter, Dr., D-69469 Weinheim (DE); Gaa, Peter, D-67551 Worms (DE)

(56) Entgegenhaltungen:
- EP-A- 0 053 696
- EP-A- 0 167 996

## Beschreibung

Die vorliegende Erfindung betrifft ein Verfahren zur Herstellung von aromatischen Aminen aus aliphatischen Aminen sowie deren Herstellung durch aminierende Hydrierung an Edelmetallkatalysatoren.

Aus der US-A-5 072 044 ist die Dehydrierung von Cyclohexylaminen zu den entsprechenden aromatischen Aminen an Li-dotierten Pd-Katalysatoren bekannt. Die Umsetzung benötigt hohe Reaktionstemperaturen von 360 bis 380°C, die die Bildung von unerwünschten Nebenprodukten fördern.

Die einstufige Überführung von Phenolen mit Ammoniak und Wasserstoff in Cyclohexylamine in Gegenwart von Edelmetallkatalysatoren ist z.B. aus EP-A-22 751 und EP-A-53 891 bekannt. Dabei wird fast ausschließlich die Bildung der gesättigten cyclischen Amine beobachtet, während aromatische Amine kaum entstehen.

Das EP-A-167 996 beschreibt die direkte Herstellung von aromatischen Aminen in einer zweistufigen Synthese in einem Reaktor mit zwei Reaktionszonen bzw. in zwei hintereinandergeschalteten Reaktoren. Hierbei wird in der ersten Stufe bei niedrigen Temp. (180 bis 210°C) die aminierende Hydrierung zum Cyclohexylamin und in der zweiten Stufe, ohne vorherige Aufarbeitung, bei höheren Temperaturen (220 bis 250°C) die Dehydrierung zum entsprechenden aromatischen Amin durchgeführt. Eine Beschreibung geeigneter Katalysatoren findet sich beispielsweise in der DE-A-32 09 148, der EP-A-53 696, der EP-A-53 817 oder EP-A-53 818. Im längeren Betrieb zeigt sich aber, daß der in der 2. Stufe eingesetzte Pd-Katalysator, der 1 Gew.-% Pd auf einem Träger von 19,4 Gew.-% MgO und 80,6 Gew.-% Al₂O₃ enthält, wesentlich schneller desaktiviert als der Katalysator der 1. Stufe, so daß die Anlage häufig zum Katalysatorwechsel bzw. -regeneration abgestellt werden muß.

Der vorliegenden Erfindung lag daher die Aufgabe zugrunde, den zuvor genannten Nachteilen abzuhelfen.

Demgemäß wurde ein neues und verbessertes Verfahren zur Herstellung von aromatischen Aminen der allgemeinen Formel I 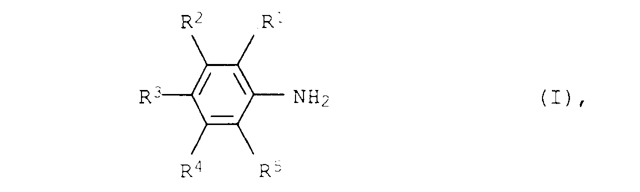 in der R¹, R², R³, R⁴ und R⁵ für Wasserstoff, C₁- bis C₁₂-Alkyl oder C₃- bis C₁₂-Cycloalkyl stehen, aus aliphatischen Aminen der allgemeinen Formel II 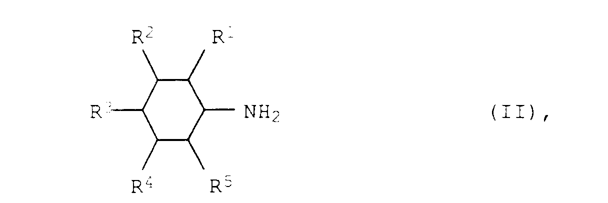 in der R¹, R², R³, R⁴ und R⁵ die obengenannten Bedeutungen haben, bei Temperaturen von 150 bis 300°C und Drücken von 0,01 bis 50 bar in Gegenwart eines Heterogenkatalysators gefunden, welches dadurch gekennzeichnet ist, daß der Heterogenkatalysator aus 30 bis 100 Gew.-%
a) Palladium auf Oxiden der seltenen Erden und/oder auf Oxiden der IV. Nebengruppe oder
b) Platin/Palladium-Gemischen auf Aluminiumoxid und/oder Oxiden der seltenen Erden und/oder Oxiden der IV. Nebengruppe
und 0 bis 70 Gew.-% Alkali- und/oder Erdalkalioxid besteht sowie die Herstellung der aliphatischen Amine der Formel II durch Umsetzung von Phenolen der allgemeinen Formel III 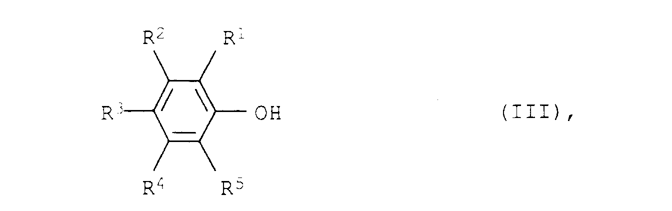 in der R¹, R², R³, R⁴ und R⁵ die obengenannten Bedeutungen haben, mit Ammoniak und Wasserstoff bei Temperaturen von 100 bis 250°C und Drücken von 0,01 bis 50 bar in Gegenwart eines obengenannten Katalysators.

Das erfindungsgemäße Verfahren läßt sich wie folgt durchführen:
Die Umsetzung von den aliphatischen Aminen II zu den aromatischen Aminen I kann an einem Heterogenkatalysator in der Flüssigphase, bevorzugt in der Gasphase bei Temperaturen von 150 bis 300°C, bevorzugt 170 bis 270°C, besonders bevorzugt 180 bis 250°C und Drücken von 0,01 bis 50 bar, bevorzugt 0,1 bis 5 bar, besonders bevorzugt Normaldruck (Atmosphärendruck) durchgeführt werden.

Die Umsetzung von den Phenolen III mit Ammoniak und Wasserstoff zu den aliphatischen Aminen II kann an einem Heterogenkatalysator in der Flüssigphase, bevorzugt in der Gasphase bei Temperaturen von 100 bis 250°C, bevorzugt 120 bis 220°C, besonders bevorzugt 140 bis 200°C und Drücken von 0,01 bis 50 bar, bevorzugt 0,1 bis 5 bar, besonders bevorzugt Normaldruck (Atmosphärendruck) durchgeführt werden.

Ammoniak und Wasserstoff werden - bezogen auf das eingesetzte substituierte Phenol - in der Regel in einem Molverhältnis von 2:1 bis 200:1, bevorzugt 3:1 bis 100:1, besonders bevorzugt 4:1 bis 40:1 eingesetzt. Das Molverhältnis von Ammoniak zu Wasserstoff kann 100:1 bis 0,01:1, bevorzugt 10:1 bis 0,1:1, besonders bevorzugt 5:1 bis 0,5:1 betragen.

Die beiden zuvor beschriebenen Reaktionen können nacheinander in zwei Reaktionszonen z.B. in einem Rohrreaktor durchgeführt werden.

Im Prinzip kommt es aber nur darauf an, eine Rückvermischung vom Ausgang der Anlage, d.h. dem Ende der letzten Reaktionszone zum Eingang, d.h. dem Beginn der ersten Reaktionszone zu vermeiden, was sich in Rohrreaktoren und der sich darin ausbildenden Pfropfenströmung am leichtesten erreichen läßt.

Der Katalysator kann jeweils in einem Festbett oder auch im Wirbelbett angeordnet sein. Als maßgeschneiderte Katalysatoren für die zweite Reaktionszone bewähren sich die erfindungsgemäßen Heterogenkatalysatoren.

Als Heterogenkatalysatoren eignen sich solche die aus 30 bis 100 Gew.-%, bevorzugt 50 bis 100 Gew.-%, besonders bevorzugt 70 bis 100 Gew.-%
a) Palladium auf Oxiden der seltenen Erden und/oder auf Oxiden der IV. Nebengruppe oder
b) Platin/Palladium-Gemischen auf Aluminiumoxid und/oder Oxiden der seltenen Erden und/oder Oxiden der IV. Nebengruppe
und 0 bis 70 Gew.-%, bevorzugt 0 bis 50 Gew.-%, besonders bevorzugt 0 bis 30 Gew.-% Alkali- und/oder Erdalkalioxid besteht.

Als Oxide der seltenen Erden eignen sich die Elemente der Lanthaniden - und der Actinidengruppe des Periodensystems der Elemente wie Cer, Praseodym, Neodym, Promethium, Samarium, Europium, Gadolinium, Terbium, Dysprosium, Holmium, Erbium, Thulium, Ytterbium, Lutetium, Thorium, Protactinium, Uran, Neptunium, Plutonium, Americum, Curium, Berkelium, Californium, Einsteinium, Fermium, Mendelevium, Nobelium und Lawrencium, bevorzugt Cer, Praseodym, Neodym, Samarium, Europium, Terbium, Ytterbium, Thorium und Protactinium, besonders bevorzugt Cer, Praseodym, Neodym und Thorium.

Als Oxide der IV. Nebengruppe eignen sich Titan, Zirkon und Hafnium, bevorzugt Titan und Zirkon, besonders bevorzugt Zirkon.

Als Alkali- und/oder Erdalkalioxide eignen sich Lithium, Natrium, Kalium, Rubidium, Caesium, Francium, Beryllium, Magnesium, Calcium, Strontium und Barium, bevorzugt Natrium, Kalium, Magnesium, Calcium, Strontium und Barium, besonders bevorzugt Natrium, Kalium, Magnesium, Calcium und Barium.

Die aktiven Bestandteile des Katalysators (Edelmetalle) befinden sich bei reinem Palladium vorzugsweise auf Oxiden der seltenen Erden (Lanthaniden und Actiniden) oder auf Oxiden der IV. Nebengruppe und beim Platin/Palladium vorzugsweise auf im wesentlichen aus Aluminiumoxid aufgebauten Trägern, auf Oxiden der seltenen Erden oder auf Oxiden der IV. Nebengruppe.

Die Katalysatoren können entweder durch gemeinsames Kneten der Zusätze (in Form der Metalloxide) mit Aluminiumoxid, thermische Nachbehandlung (Tempern) bei 400 bis 900°C und Tränken mit einer das Edelmetall enthaltenden Lösung oder durch Tränken des Trägers mit einer Lösung der Zusätze und des Hydriermetalls, z.B. in Form von Lösungen ihrer Nitrate, Chloride, Formiate, Oxalate oder Ammoniakate und darauf folgendes Tempern bei 400 bis 900°C hergestellt werden. Bei der Spinellbildung muß nach dem Kneten bzw. Tränken des Aluminiumoxids mit dem Oxid bzw. der Lösung der Zusatzkomponente eine Temperatur von 900 bis 1 300°C erreicht werden (siehe Ullmanns Encyklopädie der technischen Chemie, 3. Auflage (1955) Band 6, Seite 242 bis 244, Gmelin, System-Nr. 35, Al Tl 1934 bis 1995, Seite 26 bis 28).

Der Edelmetallgehalt des Katalysators, bezogen auf das Trägermaterial, liegt in der Regel bei 0,0001 bis 25 Gew.-%, bevorzugt 0,001 bis 20 Gew.-%, besonders bevorzugt 0,05 bis 15 Gew.-%. Man kann die Katalysatoren beispielsweise in Form von Formkörpern, z.B. Strängen oder als Pulver, je nach vorgesehenem Verwendungszweck einsetzen.

Das erfindungsgemäße Verfahren hat gegenüber den bekannten Verfahren den Vorteil, daß durch den Einsatz von aktiveren und langsamer desaktivierenden Katalysatoren im Dehydrierschritt die Abstellungen zum Katalysatorwechsel im technischen Betrieb deutlich minimiert werden und dadurch die Kapazität erhöht wird. Darüber hinaus haben die entwickelten Katalysatoren eine höhere Anfangsselektivität zum aromatischen Amin, so daß die Selektivität über die gesamte Laufzeit entsprechend besser ist.

Die Bildung von normalerweise typischen Nebenprodukten, wie Cyclohexanolen, Dicyclohexylaminen, Diphenylaminen u.a. kann bei diesem Verfahren weitgehend unterdrückt werden, so daß keine oder nur Spuren dieser Nebenprodukte auftreten.

Die Substituenten R¹, R², R³, R⁴ und R⁵ in den Verbindungen I, II und III haben folgende Bedeutungen:
- Wasserstoff,
- C₁- bis C₁₂-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₁₂-Cycloalkyl, bevorzugt C₅- bis C₈-Cycloalkyl, besonders bevorzugt C₅- bis C₆-Cycloalkyl wie Cyclopentyl und Cyclohexyl.

Alle mit inerten Substituenten substituierte Phenole III können für das erfindungsgemäße Verfahren eingesetzt werden, beispielsweise ortho-, meta- und para-Kresol, ortho-Ethylphenol, ortho-n-Butylphenol, ortho-sec.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-cyclohexyl-phenol, 2,6-Diethylphenol, 2,5-Diisopropylphenol, 2-Methyl-6-sec.-butylphenol, 3-tert.-Butylphenol, 2,6-Di-sec.-Butylphenol, 2,6-Dicyclohexylphenol.
Die nach dem Verfahren der Erfindung hergestellten Verbindungen werden z.B. zur Herstellung von Wirkstoffen in Pflanzenschutzmitteln verwendet (DE-A-23 05 495, DE-A-26 48 008, DE-A-25 13 732, DE-A-25 15 091).

### Beispiele

### Beispiel 1 (Vergleichsbeispiel):

8 kg Aluminiumoxid werden mit 2 kg Magnesiumoxid (jeweils berechnet auf reines Al₂O₃ und MgO) unter Zugabe von ca. 10 Litern Wasser geknetet und anschließend im Extruder zu 4 mm Strängen verformt. Die erhaltenen Stränge werden bei 120°C 6 h getrocknet und danach bei 450°C 2 h getempert.

Die Stränge werden in einer Imprägniertrommel mit einer Pd-nitratlösung derart getränkt, daß die 5 Gew.-%ige Lösung heiß auf die Stränge gesprüht wird. Die Stränge werden dann bei 120°C 4 h getrocknet und danach bei 520°C 2 h calziniert.

Der so hergestellte Katalysator A (analog EP-A-167 996) enthält 1 Gew.-% Pd auf einen Träger, der aus 19,4 Gew.-% Magnesiumoxid und 80,6 Gew.-% Aluminiumoxid besteht.

In ein elektrisch beheizbares 2,5 l fassendes Reaktorrohr von 1 m Länge werden 750 ml Katalysator eingefüllt. Der Reaktor wird dann im Stickstoff (100l/h) auf 180°C (5°C/min) aufgeheizt und dabei gehalten. Während der nächsten 4 h wird 30 l/h Wasserstoff zudosiert. Anschließend wird der Stickstoff über 4 Stunden zurückgenommen, bis nur noch Wasserstoff durch den Reaktor gefahren wird. Dann wird die Temperatur zunächst auf 200°C vorgefahren und 2 h gehalten und anschließend auf 220°C erhöht und erneut 2 h gehalten. Der aktivierte Katalysator wird direkt für den Versuch eingesetzt und bis Versuchsbeginn unter Stickstoff gehalten.

Zur Reaktion wird unter Normaldruck im NH₃(90 l/h)/H₂(210 l/h) - Strom auf Reaktionstemperatur gebracht. Die Temperatur im vorgeschalteten Verdampfer wird auf den gleichen Wert eingestellt. Zum Gasstrom werden 100 ml/h Zulauf über den Verdampfer eindosiert. Nach dem Reaktor werden die flüssigen Reaktionsprodukte über einen zweistufigen Intensivkühler mit nachgeschalteter Kühlfalle auskondensiert und gaschromatographisch analysiert. Nach Ende des Versuchstages wird noch 1 h mit Ammoniak/Wasserstoff gespült und schließlich unter Stickstoff abgekühlt.

Der Zulauf für die Versuche zum Dehydrierschritt wird über mehrere Wochen in der Apparatur bei 180°C analog EP-A-167 996 hergestellt. Das hergestellte Dimethylcyclohexylamin hat folgende Zusammensetzung.

| | |
|---|---|
| 2,7 % m-Xylol | |
| 86,0 % Dimethylcyclohexylamin | (DMCHA) |
| 3,7 % Dimethylphenol | (DMP) |
| 7,3 % Dimethylanilin | (Xylidin) |
| 4,9 % Wasser | (Karl-Fischer-Titration) |

Am aktivierten Katalysator wird der Zulauf tageweise dehydriert. Nach Zulaufende wird der Reaktor im Stickstoffstrom abgekühlt und der Katalysator über Nacht unter Stickstoff gehalten. Wenn der Xylidingehalt unter 60 % (GC-Flächenprozent) sinkt, wird die Reaktionstemperatur um 10°C erhöht. Die Ergebnisse zeigt nachfolgende Tabelle.

| Dauer [d] | Temp. [8°C] | Xylidin [%] | DMCHA/O [%] | m-Xylol [%] | DMP [%] |
|---|---|---|---|---|---|
| 2 | 220 | 74,6 | 21,2 | 3 | 0,1 |
| 8 | 230 | 62,6 | 33,4 | 2,8 | 0,4 |
| 12 | 240 | 61,6 | 33,4 | 3,3 | 0,9 |
| 15 | 250 | 61,7 | 30 | 3,9 | 11,5 |

Man erkennt die schnelle Desaktivierung des Katalysators, die durch häufiges Vorfahren der Temperatur ausgeglichen werden muß.

### Beispiel 2

Man verfährt entsprechend Beispiel 1, verwendet aber den Katalysator C. Katalysator C wird entsprechend Katalysator A hergestellt, jedoch wird anstelle der Pd-nitrat Lösung eine Lösung verwendet, die in gleichen Mengen Pd-nitrat und Pt-nitrat enthält. Der so hergestellte Katalysator enthält 0.5 Gew.-% Pt und 0.5 Gew.-% Pd.

| Dauer [d] | Temp. [8°C] | Xylidin [%] | DMCHA/O [%] | m-Xylol [%] | DMP [%] |
|---|---|---|---|---|---|
| 3 | 210 | 71,4 | 21 | 5,1 | 1,1 |
| 10 | 210 | 64,1 | 28,4 | 5,1 | 1,2 |
| 20 | 220 | 76,6 | 13 | 7 | 2,2 |
| 40 | 220 | 71,8 | 21,6 | 4,4 | 1,1 |

Man erkennt, daß dieser Katalysator sowohl sehr langsam desaktiviert als auch wenig Nebenprodukte liefert.

### Beispiel 3

Man verfährt entsprechend Beispiel 1, verwendet aber den Katalysator D.

6 kg frisch gefälltes Cerhydroxid werden mit 60 g Salpetersäure und 120 g Wasser 3 h geknetet und anschließend im Extruder zu 4 mm Strängen verformt. Die erhaltenen Stränge werden bei 120°C 16 h getrocknet und danach bei 520°C 3 h getempert.

Die Stränge werden in einer Imprägniertrommel mit einer Pd-nitratlösung derart getränkt, daß die 5 Gew.-%ige Lösung heiß auf die Stränge gesprüht wird. Die Stränge werden dann bei 120°C 4 h getrocknet und danach bei 520°C 2 h calziniert.

Der so hergestellte Katalysator enthält 1 Gew.-% Pd.

| Dauer [d] | Temp. [8°C] | Xylidin [%] | DMCHA/O [%] | m-Xylol [%] | DMP [%] |
|---|---|---|---|---|---|
| 2 | 220 | 86, 7 | 4,3 | 5,5 | 1,2 |
| 10 | 220 | 74,3 | 16 | 5,2 | 3 |
| 20 | 220 | 68,6 | 25,9 | 3,3 | 1,3 |
| 40 | 230 | 73 | 19,7 | 4 | 2,1 |

Man erkennt, daß dieser Katalysator eine sehr hohe Anfangsaktivität hat und sehr langsam desaktiviert.

### Beispiel 4

Man verfährt entsprechend Beispiel 1, verwendet aber den Katalysator F. Katalysator F wird entsprechend Katalysator A hergestellt, jedoch werden ZrO₂-Stränge und anstelle der Pd-nitrat-Lösung eine Lösung verwendet, die in gleichen Mengen Pd-nitrat und Pt-nitrat enthält. Der so hergestellte Katalysator enthält 0,5 Gew.-% Pt und 0,5 Gew.-% Pd.

| Dauer [d] | Temp. [8°C] | Xylidin [%] | DMCHA/O [%] | m-Xylol [%] | DMP [%] |
|---|---|---|---|---|---|
| 3 | 210 | 72,4 | 19,6 | 5,2 | 0,1 |
| 8 | 210 | 70,7 | 23,0 | 4,4 | 0,1 |
| 10 | 210 | 68,8 | 25,0 | 4,2 | 0,1 |

Man erkennt, daß dieser Katalysator eine hohe Aktivität hat und sehr langsam desaktiviert.

## Patentansprüche

1. Verfahren zur Herstellung von aromatischen Aminen der allgemeinen Formel I 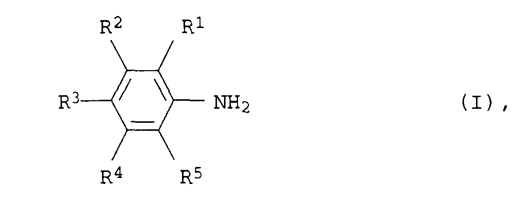 in der R¹, R², R³, R⁴ und R⁵ für Wasserstoff, C₁- bis C₁₂-Alkyl oder C₃- bis C₁₂-Cycloalkyl stehen, aus aliphatischen Aminen der allgemeinen Formel II 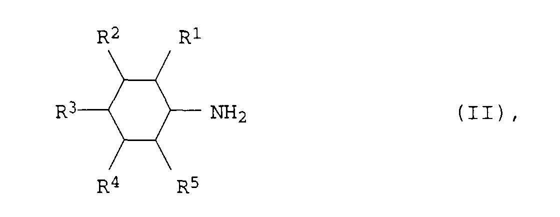 in der R¹, R², R³, R⁴ und R⁵ die obengenannten Bedeutungen haben, bei Temperaturen von 150 bis 300°C und Drücken von 0,01 bis 50 bar in Gegenwart eines Heterogenkatalysators, dadurch gekennzeichnet, daß der Heterogenkatalysator aus 30 bis 100 Gew.-%
a) Palladium auf Oxiden der seltenen Erden und/oder auf Oxiden der IV. Nebengruppe oder
b) Platin/Palladium-Gemischen auf Aluminiumoxid und/oder Oxiden der seltenen Erden und/oder Oxiden der IV. Nebengruppe
und 0 bis 70 Gew.-% Alkali- und/oder Erdalkalioxid besteht.

2. Verfahren zur Herstellung von aromatischen Aminen der Formel I nach Anspruch 1, dadurch gekennzeichnet, daß man die aliphatischen Amine der Formel II durch Umsetzung von Phenolen der allgemeinen Formel III 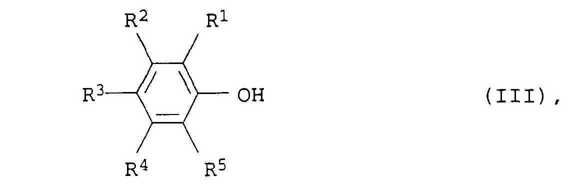 in der R¹, R², R³, R⁴ und R⁵ die obengenannten Bedeutungen haben, mit Ammoniak und Wasserstoff bei Temperaturen von 100 bis 250°C und Drücken von 0,01 bis 50 bar in Gegenwart eines Katalysators von Anspruch 1 durchführt.

3. Verfahren zur Herstellung von aromatischen Aminen der Formel I nach Anspruch 1, dadurch gekennzeichnet, daß man Heterogenkatalysatoren einsetzt, die aus 50 bis 100 Gew.-%
a) Palladium auf Oxiden der seltenen Erden und/oder auf Oxiden der IV. Nebengruppe oder
b) Platin/Palladium-Gemischen auf Aluminiumoxid und/oder Oxiden der seltenen Erden und/oder Oxiden der IV. Nebengruppe
und 0 bis 50 Gew.-% Alkali- und/oder Erdalkalioxid bestehen.

4. Verfahren zur Herstellung von aromatischen Aminen der Formel I nach Anspruch 1, dadurch gekennzeichnet, daß man Heterogenkatalysatoren einsetzt, die aus 70 bis 100 Gew.-%
a) Palladium auf Oxiden der seltenen Erden und/oder auf Oxiden der IV. Nebengruppe oder
b) Platin/Palladium-Gemischen auf Aluminiumoxid und/oder Oxiden der seltenen Erden und/oder Oxiden der IV. Nebengruppe
und 0 bis 30 Gew.-% Alkali- und/oder Erdalkalioxid bestehen.

5. Verfahren zur Herstellung von aromatischen Aminen der Formel I nach Anspruch 1, dadurch gekennzeichnet, daß man Heterogenkatalysatoren einsetzt, die als Oxid der selten Erden aus 30 bis 100 Gew.-% CeO₂ bestehen.

6. Verfahren zur Herstellung von aromatischen Aminen der Formel I nach Anspruch 1, dadurch gekennzeichnet, daß man Heterogenkatalysatoren einsetzt, die als Oxid der IV. Nebengruppe aus 30 bis 100 Gew.-% ZrO₂ bestehen.

7. Verfahren zur Herstellung von aromatischen Aminen der Formel I nach Anspruch 1, dadurch gekennzeichnet, daß die Substituenten R¹, R², R³, R⁴ und R⁵ für Wasserstoff, C₁- bis C₈-Alkyl oder C₅- bis C₈-Cycloalkyl stehen.

8. Verfahren zur Herstellung von aromatischen Aminen der Formel I nach Anspruch 1, dadurch gekennzeichnet, daß die Substituenten R¹, R², R³, R⁴ und R⁵ für Wasserstoff, C₁- bis C₄-Alkyl oder C₅- oder C₆-Cycloalkyl stehen.

## Claims

1. A process for the preparation of aromatic amines of the formula I 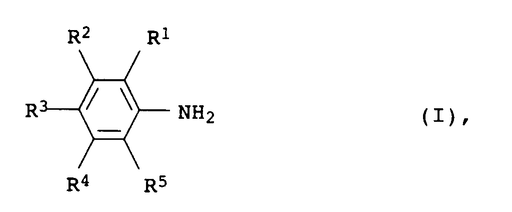 where R¹, R², R³, R⁴ and R⁵ are each hydrogen, C₁-C₁₂-alkyl or C₃-C₁₂-cycloalkyl, from aliphatic amines of the formula II 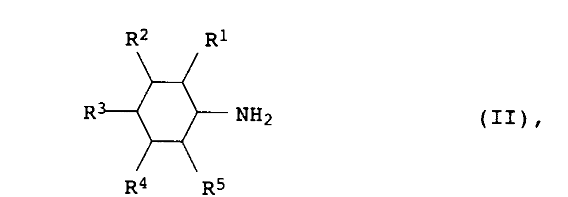 where R¹, R², R³, R⁴ and R⁵ have the abovementioned meanings, at from 150 to 300°C and from 0.01 to 50 bar in the presence of a heterogeneous catalyst, wherein the heterogeneous catalyst consists of from 30 to 100 % by weight of
a) palladium on oxides of the rare earth elements or on oxides of subgroup IV or
b) platinum/palladium mixtures on alumina or oxides of the rare earth elements or oxides of subgroup IV and from 0 to 70 % by weight of alkali metal or alkaline earth metal oxide.

2. A process for the preparation of aromatic amines of the formula I as claimed in claim 1, wherein the aliphatic amines of the formula II are prepared by reacting phenols of the formula III 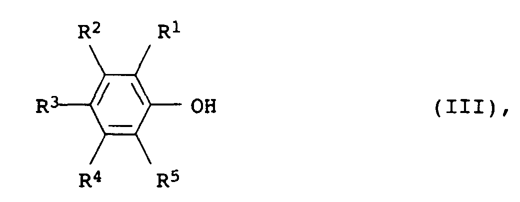 where R¹, R², R³, R⁴ and R⁵ have the abovementioned meanings, with ammonia and hydrogen at from 100 to 250°C and from 0.01 to 50 bar in the presence of a catalyst of claim 1.

3. A process for the preparation of aromatic amines of the formula I as claimed in claim 1, wherein a heterogeneous catalyst which consists of from 50 to 100 % by weight of
a) palladium on oxides of the rare earth elements or on oxides of subgroup IV or
b) platinum/palladium mixtures on alumina or oxides of the rare earth elements or oxides of subgroup IV
and from 0 to 50 % by weight of alkali metal or alkaline earth metal oxide is used.

4. A process for the preparation of aromatic amines of the formula I as claimed in claim 1, wherein a heterogeneous catalyst which consists of from 70 to 100 % by weight of
a) palladium on oxides of the rare earth elements or on oxides of subgroup IV or
b) platinum/palladium mixtures on alumina or oxides of the rare earth elements or oxides of subgroup IV
and from 0 to 30 % by weight of alkali metal or alkaline earth metal oxide is used.

5. A process for the preparation of aromatic amines of the formula I as claimed in claim 1, wherein a heterogeneous catalyst which consists of from 30 to 100 % by weight of CeO₂ as an oxide of the rare earth elements is used.

6. A process for the preparation of aromatic amines of the formula I as claimed in claim 1, wherein a heterogeneous catalyst which consists of from 30 to 100 % by weight of ZrO₂ as an oxide of subgroup IV is used.

7. A process for the preparation of aromatic amines of the formula I as claimed in claim 1, wherein R¹, R², R³, R⁴ and R⁵ are each hydrogen, C₁-C₈-alkyl or C₅-C₈-cycloalkyl.

8. A process for the preparation of aromatic amines of the formula I as claimed in claim 1, wherein R¹, R², R³, R⁴ and R⁵ are each hydrogen, C₁-C₄-alkyl or C₅- or C₆-cycloalkyl.

## Revendications

1. Procédé de préparation d'amines aromatiques de la formule générale I : 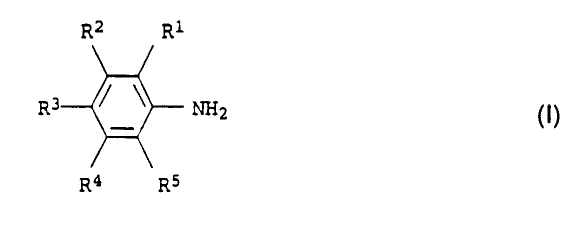 dans laquelle R¹, R², R³, R⁴ et R⁵ représentent chacun un atome d'hydrogène, un radical alkyle en C₁ à C₁₂ ou cycloalkyle en C₃ à C₁₂, à partir d'amines aliphatiques de la formule générale II : 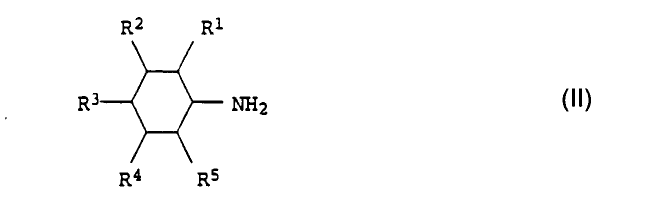 dans laquelle R¹, R², R³, R⁴ et R⁵ possèdent les significations qui leur ont été attribuées ci-dessus, à des températures de 150 à 300°C et sous des pressions de 0,01 à 50 bars, en présence d'un catalyseur hétérogène, caractérisé en ce que le catalyseur hétérogène se compose de 30 à 100% en poids
a) de palladium sur des oxydes des terres rares et/ou des oxydes des éléments du IVème sous-groupe, ou
b) de mélanges de platine/palladium sur de l'oxyde d'aluminium et/ou des oxydes des terres rares et/ou des oxydes des éléments du IVème sous-groupe,
et de 0 à 70% en poids d'un hydroxyde de métal alcalin et/ou de métal alcalino-terreux.

2. Procédé de préparation d'amines aromatiques de la formule suivant la revendication 1, caractérisé en ce que l'on prépare les amines aliphatiques de la formule II par réaction de phénols de la formule générale III : 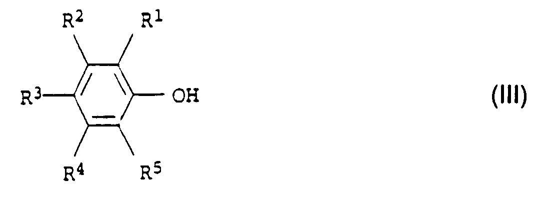 dans laquelle R¹, R², R³, R⁴ et R⁵ possèdent les significations qui leur ont été précédemment attribuées, avec de l'ammoniac et de l'hydrogène à des températures de 100 à 250°C et sous des pressions de 0,01 à 50 bars, en présence d'un catalyseur suivant la revendication 1.

3. Procédé de préparation d'amines aromatiques de la formule suivant la revendication 1, caractérisé en ce que l'on utilise des catalyseurs hétérogènes, qui se composent de 50 à 100% en poids
a) de palladium sur des oxydes des terres rares et/ou des oxydes des éléments du IVème sous-groupe, ou
b) de mélanges de platine/palladium sur de l'oxyde d'aluminium et/ou des oxydes des terres rares et/ou des oxydes des éléments du IVème sous-groupe,
et de 0 à 50% en poids d'un hydroxyde de métal alcalin et/ou de métal alcalino-terreux.

4. Procédé de préparation d'amines aromatiques de la formule suivant la revendication 1, caractérisé en ce que l'on utilise des catalyseurs hétérogènes, qui se composent de 70 à 100% en poids
a) de palladium sur des oxydes des terres rares et/ou des oxydes des éléments du IVème sous-groupe, ou
b) de mélanges de platine/palladium sur de l'oxyde d'aluminium et/ou des oxydes des terres rares et/ou des oxydes des éléments du IVème sous-groupe,
et de 0 à 30% en poids d'un hydroxyde de métal alcalin et/ou de métal alcalino-terreux.

5. Procédé de préparation d'amines aromatiques de la formule suivant la revendication 1, caractérisé en ce que l'on utilise des catalyseurs hétérogènes qui, à titre d'oxyde des terres rares, se composent de 30 à 100% en poids de CeO₂.

6. Procédé de préparation d'amines aromatiques de la formule suivant la revendication 1, caractérisé en ce que l'on utilise des catalyseurs hétérogènes qui, à titre d'oxyde d'un élément du IVème sous-groupe, se composent de 30 à 100% en poids de ZrO₂.

7. Procédé de préparation d'amines aromatiques de la formule suivant la revendication 1, caractérisé en ce que les symboles R¹, R², R³, R⁴ et R⁵ représentent chacun un atome d'hydrogène, un radical alkyle en C₁ à C₈ ou un radical cycloalkyle en C₅ à C₈.

8. Procédé de préparation d'amines aromatiques de la formule suivant la revendication 1, caractérisé en ce que les symboles R¹, R², R³, R⁴ et R⁵ représentent chacun un atome d'hydrogène, un radical alkyle en C₁ à C₄ ou un radical cycloalkyle en C₅ ou C₈.
